# EUROPÄISCHE PATENTSCHRIFT

(11) **EP 2 536 400 B1**
(45) Veröffentlichungstag und Bekanntmachung des Hinweises auf die Patenterteilung: **24.09.2014**
(21) Anmeldenummer: 11708184.4
(22) Anmeldetag: 15.02.2011
(51) Int. Cl.: A61K 31/131, A61K 31/14, A61K 31/16, A61K 45/06

(54) **ARZNEIMITTEL ENTHALTEND MYRAMISTIN**
MEDICAMENT COMPRISING MYRAMISTIN
MÉDICAMENT COMPRENANT DE LA MYRAMISTINE

(30) Priorität: 19.02.2010 WO PCT/EP2010/001056
(43) Veröffentlichungstag der Anmeldung: 26.12.2012
(73) Patentinhaber: Megainpharm GmbH, 9082 Maria Wörth (AT)
(72) Erfinder: RUDKO, Adolina, Kiev 252114 (UA)
(74) Vertreter: Jeck, Anton
(86) Internationale Anmeldenummer: PCT/EP2011/000691
(87) Internationale Veröffentlichungsnummer: WO 2011/101113

(56) Entgegenhaltungen:
- EP-A1- 1 634 590
- WO-A1-93/00892
- WO-A2-2007/136558
- DE-A1-102008 039 254
- RU-C1- 2 161 961
- RU-C2- 2 157 214
- SU-A1- 1 796 185
- UA-C2- 64 800
- UA-C2- 67 795
- US-A- 2 459 062
- US-A- 4 093 711
- US-A1- 2004 058 924

## Beschreibung

Die Erfindung betrifft das Gebiet der Medizin und die Pharmaindustrie. Sie kann bei der Entwicklung, Herstellung und Anwendung von Heil- und vorbeugenden Präparaten für Menschen und Tiere benutzt werden.

Die Synthese und die Anwendung von quartären Ammoniumsalzen, die gleichzeitig eine Amidgruppe enthalten, sind erstmals im US-Patent 2459062 (D1) beschrieben. Insbesondere im Beispiel 1 ist die Synthese von [Myristamido]-propyl)-dimethylbenzyl-ammoniumchlorid durch die Reaktion von [(Myris-tamido-)propyl]-Dimethylamin mit Benzylchlorid im Benzol beschrieben.

In diesem Patent ist die Aktivität des hergestellten Produkts in Bezug auf den Standardstamm von Staf gezeigt. Das von den Erfindern beschriebene Produkt stellt bei Raumtemperatur einen halbharten Stoff mit einer Schmelztemperatur von 54°C (Spalte 3, Zeilen 69 bis 70) dar. Dabei werden im Beispiel 1 keine anderen Untersuchungen angegeben, welche die Struktur des erhaltenen Produkts bestätigen würden. Aber die erfindungsgemäßen Untersuchungen in Bezug auf die Absonderung eines reinen, für die medizinische Anwendung geeigneten Präparates zeigen, dass [Myristamido]-propyl)-dimethylbenzyl-ammoniumchlorid bei Raumtemperatur einen kristallischen Stoff mit einer Schmelztemperatur von über 90°C darstellt.

Somit kann angenommen werden, dass die Erfinder die Mischung aus dem Hauptprodukt und dem Rückstand aus Benzylchlorid und dem Lösungsmittel (Benzol) erhalten und deren Wirksamkeit untersucht haben. Dennoch ist die Anwendung eines solchen Produkts für medizinische Zwecke aufgrund der hohen Toxizität von Benzylchlorid und Benzol nicht möglich.

Gemäß Spalte 3, Zeile 62 ist das grundlegende [(Myristamido-)propyl]-dimethylamin auch ein Feststoff, was auch von den erfindungsgemäßen Untersuchungen bewiesen wird. Aus diesem Grund kann das im Beispiel 1 erhaltene Produkt keine Mischung aus dem grundlegenden Amidoamin und der Endverbindung darstellen. Außerdem ist das Vörhandensein der Rückstände aus dem grundlegenden Amidoamin nicht möglich, denn im Beispiel 1 ist eine ausreichende Menge von Benzylchlorid in der Reaktion benutzt.

Ferner sei erwähnt, dass das reine [Myristamido]-propyl)-dimethylbenzylammoniumchlorid im Wasser keine Lösung mit einer Konzentration von über 20% ergibt und dass das grundlegende [(Myristamido-)propyl]-dimethylamin sich im Wasser überhaupt nicht auflöst und mittels Wasser aus anderen Lösungen in anderen Lösungsmitteln ausgeschieden wird. Dies gilt ebenfalls als Beweisgrund dafür, dass das Endprodukt nach Beispiel 1 keine Beimischung aus [(Myristamido-)propyl]-dimethylamin enthalten kann, weil in Spalte 3, Zeilen 73 bis 74 auf die Herstellung der transparenten 25%igen Wasserlösungen hingewiesen wird.

Es ist eine Reihe von Arzneimitteln auf der Basis von Benzyldimethyl(3-[myristoylamino]-propyl)-ammoniumchlorid in Form von Monohydrat oder in nichthydratisierter Form bekannt. Diese Arzneimittel dienen zur Behandlung und Vorbeugung von bestimmten Arten von Krankheiten (SU 1796185 (D2), EP 1634590 (D3), WO 93/00892 (D4), RU 2157214 (D5), UA 67795 (D6), UA 64800 (D7), RU 2161961 (D8), RU 2188005 (D13), RU 2184534 (D14), RU 2164135 (D15), RU 2177314 (D16), RU 2185157 (D17), RU 2185156 (D18), RU 2173142 (D19), UA 30143 (D20)). Der Mangel dieser Arzneimittel besteht entweder in einem sehr begrenzten Wirkungsbereich der jeweiligen Lösung oder in ungenügender Effizienz bei anderweitiger Anwendung oder in einer niedrigeren Effizienz im Vergleich zur vorgeschlagenen technischen Lösung. Einige konkrete, jedoch nicht ausschließliche Vergleichsangaben sind in den Beispielen der vorliegenden Beschreibung angeführt.

Weitere Vorschläge sehen die Verwendung von tertiären Aminen in den biologisch aktiven Verbindungen vor. Diese Amine enthalten die Amidgruppe, einschließlich Dimethyl[3-(myristoylamino]-propyl]amin ([(myristamido-)propyl]-dimethylamin) (z. B. US WO 2007/136558) (D10). Solche Verbindungen in der Zusammensetzung mit Polyhexametylen-Biguanidin werden in Mehrkomponenten-Zusammensetzungen zur Desinfizierung verschiedener Oberflächen verwendet. Sie können aber bei solchen Konzentrationen nicht als Arzneimittel wegen der hohen Toxizität angewendet werden. US 2004/0058924 (D9) zeigt die Möglichkeit, die Amidoamine in den Zusammensetzungen zur Behandlung einer reinen Pilzinfektion der Augen sowie deren Kombination mit Acanthamoeba einzusetzen. Im US-Patent 2004/0033208 (D21) wird vorgeschlagen, die Amidoamine in einer Zusammensetzung mit Antibiotika zur Behandlung und Vorbeugung von Pilzerkrankungen der Augen und der Nase anzuwenden. Diese Zusammensetzungen weisen aber eine niedrige Effizienz bei bakteriellen Infektionen auf und können nicht zur Behandlung von inneren Organen oder zum Beispiel als spermizide Präparate angewendet werden.

In WO95/08266 (D22) wird vorgeschlagen, die Amidoamine solchen Typs in entsprechenden Zusammensetzungen für die Reinigung der Kontaktlinsen einzusetzen. Diese Zusammensetzungen können aber nicht zur Behandlung von Erkrankungen von Augen oder z. B. von Ohren und Hals angewendet werden. Sie können auch nicht bei intrakavitären Operationen oder als Mittel zur Vorbeugung von Geschlechtskrankheiten eingesetzt werden, da sie mehrkomponentig sind und eine hohe Konzentration der Komponenten aufweisen.

Die Anwendung der Aminoxide solcher Art ist in US 4093711 (D11) beschrieben. In diesem Patent wird vorgeschlagen, die 1%igen Wasserlösungen verschiedener Aminoxide, insbesondere Dimethylaminopropyl Myristamid N-Oxids, in den Zusammensetzungen für die hygienische Behandlung der Zähne einzusetzen, um die Zahnsteinentfernung zu erleichtern und der Zahnsteinbildung vorzubeugen. In diesem Fall erfüllt das benutzte Amidoaminoxid die Funktion eines Weichungsmittels und Emulgator des Zahnbelags und ist kein Arzneimittel.

Pharmazeutische Zusammensetzungen, in denen Amidoaminoxide sowie gleichzeitig quartäres Ammoniumsalz und tertiäres Amidoamin und/oder Oxid dieses Amins verwendet werden, konnten wir den zugänglichen Literaturquellen nicht entnehmen.

Der der vorliegenden Erfindung am nächsten kommende Stand der Technik ist das im eurasischen Patent 005132 (D23) offenbarte Arzneimittel. Es besteht aus der quartären Ammoniumverbindung von Benzyldimethyl(3-[myristoylamino]-propyl)-ammoniumchlorid ([myristamido]-propyl)-dimethylbenzyl-ammoniumchlorid) in Form von Monohydrat. Als pharmazeutisches lösendes Mittel enthält dieses Arzneimittel Wasser und/oder Alkohol für flüssige Formen des Präparats. Bei anderen Darreichungsformen des Arzneimittels enthält es einen beliebigen Grundstoff pflanzlicher, tierischer oder synthetischer Herkunft. Das Präparat weist eine gute antimikrobielle sowie antimykotische Effizienz auf. Die antibakterielle Wirkung des Arzneimittels während der "in vitro"-Versuche war ziemlich hoch. Sie nimmt jedoch bei der Anwendung verschiedener Kompositionen zur Vorbeugung und Behandlung "in vivo" unter der Einwirkung einer hohen Proteinbelastung wesentlich ab. Eine wesentliche Konzentrationserhöhung verursacht andererseits die Verstärkung der Reizwirkung. Außerdem kann dieses Arzneimittel mit manchen Grundstoffen und Arzneimitteln anderer bestimmungsgemäßen Wirkung nicht wirksam kombiniert werden, weil dabei komplexe Verbindungen gebildet werden, die sich absetzen.

Erfindungsgemäß wird als Arzneimittel folgende Zusammensetzung vorgeschlagen:
Benzyldimethyl(3-[myristoylamino]-propyl)-ammoniumchlorid in Form von Monohydrat bzw. in nichthydratisierter Form mit Dimethyl(3-[myristoylamino]-propyl)-aminoxid und /oder Dimethyl(3-[myristoylamino]-propyl)-amin im geeigneten pharmazeutischen Lösungsmittel.

Für den äußerliche Gebrauch weist dabei bei zulässiger Toxizität die Zusammensetzung bei folgendem Verhältnis der Komponenten (in Gew.-%), die aus der DE 10 2008 039 254 A1 bekannt ist, die höchste Effizienz auf:

| | |
|---|---|
| Benzyldimethyl(3-[myristoylamino]-propyl)-ammoniumchlorid in Form von Monohydrat bzw, in nichthydratisierter Form | 0,008- 5,0 |
| Dimethyl(3-[myristoylamino]-propyl)-aminoxid und /oder Dimethyl(3-[myristoylamino]-propyl)-amin ein geeignetes pharmazeutisches lösendes Mittel bis 100. | 0,00005 - 1,0 |

Die niedrigste Reizwirkung und Toxizität für Menschen und Tiere bei genügender Effizienz weist die Zusammensetzung für die Anwendung an der Schleimhaut oder intrakavitäre Anwendung bei folgendem Verhältnis der Komponenten (in Gew.-%), die aus der DE 10 2008 039 254 A1 bekannt ist, auf:

| | |
|---|---|
| Benzyidimethyl(3-[myristoylamino]-propyl)-ammoniumchlorid in Form von Monohydrat bzw, in nichthydratisierter Form | 0,008- 2,0 |
| Dimethyl(3-[myristoylamino]-propyl)-aminoxid und /oder Dimethyl(3-[myristoylamino]-propyl)-amin ein geeignetes pharmazeutisches lösendes Mittel bis 100. | 0,00005 - 0,01 |

Als pharmazeutisches Lösemittel wird vorgeschlagen, Wasser und/oder Alkohol sowie einen beliebigen flüssigen, gelartigen Salben-, Wasch- oder Festgrundstoff pflanzlicher, tierischer oder synthetischer Herkunft zu verwenden, je nach der Verabreichungsweise bzw. Anwendungsstelle,

Außerdem wird eine ganze Reihe von Kombinationen dieses Arzneimittels mit Arzneimitteln anderer Wirkung vorgeschlagen. Die Arzneimittel mit anderer Wirkung umfassen zum Beispiel Lokalanästhetika (Lidocain, Bupivacain, Pyromecain oder Trimecain), Natriumchlorid, Antiödemmittel (Xylometazolin, Oxymetazolin, Naphazolin, Phenylephrin, Phenylpropanolamin oder Pseudoephedrin), Kortikosteroide (Triamcinylon, Betamethason, Fluocinolon acetonid, Hydrocortison, Halometason oder Dexamethason), Antiseptika und/oder virentötende Präparate (Metronidazol, Clotrimazol, Ketoconazol, Aciclovir oder Rimantadin).

Die vorliegende Erfindung ermöglicht es, die Vielfalt der Mittel zur Vorbeugung und Behandlung zu erweitern. Diese Mittel weisen ein weites Wirkungsspektrum und einen hohen Wirkungsgrad, eine geringe Reiz- und Giftwirkung sowie eine immunmodulatorisohe, anästhetische, antiödemische und entzündungshemmende Wirkung auf. Dies ermöglicht es, das Arzneimittel für eine größere Anzahl von Krankheiten anzuwenden. Das Präparat wird als Mittel zur Behandlung und Vorbeugung von Infektions- und Entzündungskrankheiten empfohlen. Dazu zählen auch spezifische und nicht spezifische Erkrankungen sowohl des Urogenitalsystems, des Darm- und Magenbereichs als auch Erkrankungen des Nasenrachens und der Augen. Es Ist auch als Mittel zur Vorbeugung von sexuell übertragbaren Krankheiten, wie Herpes und HIV-Infektionen, und als spermizides, antiseptisches und desinfizierendes Mittel geeignet. Erfindungsgemäß wird das Arzneimittel bei der Behandlung und Vorbeugung von eitrigen Entzündungskrankheiten mit verschiedener Ätiologie und Lokalisierung verwendet.

Die vorliegende technische Lösung hat bei der gemeinsamen Anwendung der benutzten Zutaten einen überraschenden synergistischen Effekt an den Tag gelegt, welcher durch den bekannten Stand der Technik nicht nahegelegt werden kann.

Die Wirksamkeit des vorliegenden Arzneimittels und der festgestellte Effekt werden anhand folgender Beispiele dargestellt, die allerdings nicht beschränkend sind.

### Beispiel 1

Zur Belegung der vorliegenden technischen Lösung wurden verschiedene Varianten der Zusammensetzungen des angemeldeten Präparats verwendet. In der Tabelle 1 sind einige der untersuchten Zusammensetzungen dargestellt, und zwar diejenigen, welche in den unten dargelegten Beispielen eingesetzt wurden.

**Tabelle 1**

| Varianten der Zusammensetzungen (in Ges.-%) | | | | |
|---|---|---|---|---|
| ZusammenSetzung (Z), No, | Benzyldimethyl(3-[myristoylamino]-propyl)-ammoniumchlorid (ACh) | Dimethyl[3-(myristoylamino)propyl] aminoxid (AO) | Dimethyl[3-(myristoylamino)propyl] amin (AA) | Lösungsmittel bis 100 |
| Z1 | 0,007 | 0,001 | - | Reinwasser |
| Z2 | 0,01 | 0,001 | - | isotonische Lösung NaCl |
| Z3 | 0,4* | 0,01 | 0,01 | Hydrophile Salbengrundlage |
| Z4 | 1,5 | 0,6 | - | Reinwasser |
| Z5 | 0,1 | 0,05 | - | Isotonische Lösung NaCl |
| Z6 | 0,008 | 0,001 | - | Reinwasser |
| Z7 | 0,5* | 0,01 | 0,1 | Hydrophile Salbengrundlage |
| Z8 | 0,5 | - | 0,01 | Aerosol; |
| Z9 | 0,1* | - | 0,01 | Athanol 70 % |
| Z10 | 0,04 | 0,01 | - | Reinwasser |
| Z11 | 0,05* | 0,01 | 0,01 | Suppositorien- masse |

| | | | | |
|---|---|---|---|---|
| Anmerkung:* - Monohydrat | | | | |

### Beispiel 2

Die Ermittlung von minimalen Hemmkonzentrationen (MHK) in Bezug auf eine Testkultur von Koronaviren (Koronavirus des Menschen OC43) *in vitro* an einer Nieren-Zellkultur des menschlichen Embryos. Es wurde die Zusammensetzung Z1 (zwei Komponenten im Verhältnis von 6:1) und als Prototyp zum Vergleich das Präparat nach D3 (reines Benzyldimethyl(3-[myristoylamino]-propyl)-ammoniumchlorid) und reines Dimethyl[3-(myristoylamino-)propyl] aminoxid (AA) benutzt. Die Untersuchung wurde parallel nach folgendem Schema durchgeführt:

| | |
|---|---|
| 1. Stufe | Testvirus + Myramistin (in entsprechender Konzentration) |
| 2. Stufe | Inkubation (2 Stunden) |
| 3. Stufe | Beimischung des neutralisierenden Stoffs (z. B. 25% Embryoserum der Kühe) der Zusammensetzung und Inkubation der Zusammensetzung 10 - 20 Min. |
| 4. Stufe | Ansteckung der empfindlichen Zellkulturen (z. B. Nieren des menschlichen Embryos) |
| 5. Stufe | Inkubation (5 - 8 Tage) |
| 6. Stufe | Erfassung der Ergebnisse in Bezug auf Hemadsorption und zytopathische Wirkung und Symplast-Bildung |

Es wurden je drei Reihenversuche bei vier Verdünnungen des Präparats vorgenommen. Anschließend wurden die jeweiligen MHK in Bezug auf Koronaviren ermittelt Die Ergebnisse der Versuche sind in der Tabelle 2 angeführt.

**Tabelle 2**

| MHK vom AP (angemeldeten Präparat) und anderen Antiseptika in Bezug auf Koronaviren des Menschen OC43 *in vitro* | |
|---|---|
| *Antiseptika* | MHK (%) |
| Z1 | 0,007 ± 0,002 |
| D3 (ACh) | 0,01 ± 0,005 |
| AO | 0,1 ± 0,05 |

Wie aus den in der Tabelle 2 angeführten Angaben ersichtlich ist, übertrifft Z1 in Bezug auf die Inaktivierungswirkung der Koronaviren beide Zutaten im Einzelnen und zeigt synergetischen Effekt.

### Beispiel 3

Die lokale Reizwirkung des angemeldeten Präparats auf die Haut sowie auf die Bindehaut und die Schleimhaut der Scheide wurde an Meerschweinchen und Kaninchen untersucht. Die lokale Reizwirkung auf die Schleimhaut der Harnröhre und der Harnblase wurde an Hunden untersucht.

Die erfassten Angaben haben gezeigt, dass die Anwendungen der Zusammensetzungen Z4 und Z9 auf die Haut im Laufe von 40 Tagen weder sichtbare noch histologische Änderungen ausgelöst haben. Die Erhöhung der Gesamtkonzentration der Zutaten im Präparat bis zu 5,0 Gew.-% verursachte am 30. Tag der Anwendungen eine geringe Xerodermie und eine unbedeutende Atrophie der Epidermis.

Zugleich verursachten in D8, D20 und D23 die Lösungen des reinen ACh bereits in Konzentrationen über 1 Gew.-% eine Hautreizung, wobei höhere Konzentrationen (2-3 Gew.-%) ab dem 10. - 15. Tag wesentlich stärkere Hautveränderungen herbeileiteten.

Bei der Anwendung der 0,01% igen Lösung des reinen AA in 70%igem Alkohol zeigten sich die Hautreizungen bereits am 10. Tag der Anwendung.

Die Lösung der angemeldeten Zusammensetzung (Z5) in der isotonischen Lösung Natriumchlorids wurde in die Augen von Kaninchen und Meerschweinchen im Laufe von 10 Tagen mit Gesamtkonzentrationen der Zutaten von 0,15 Gew.-% eingeträufelt. Das löste keine Reizwirkungen auf die Schleimhäute aus (Beurteilung nach der Draise-Skala = 0, Beurteilung nach der Ogur-Klassifikation = Gruppe A). Zugleich verursachte die Konzentration des Präparats nach D23 über 0,05 Gew.-% Reizwirkungen an der Bindehaut (nach der Draise-Skala = 8 bis 10 Punkte, Beurteilung nach der Ogur-Klassifikation = Gruppe B).

Je 20 ml der 0,05%igen Lösung der angemeldeten Zusammensetzung (AZ, Z10) im Wasser wurden im Laufe von 10 Tagen viermal am Tag in die Harnröhre von männlichen Hunden eingeträufelt. Nach dieser Behandlung wurden keine Veränderungen im Benehmen der Tiere beobachtet. Die Harnanalyse, die Blutprobe und die histologische Untersuchung der Schleimhaut von Harnröhre, Harnblase und anderer Organe der Hunde ergaben keine Abweichungen von der Norm.

Ebenfalls wurden keine morphologischen und histologischen Abweichungen in der Schleimhaut der Scheide der weiblichen Tiere bei der Instillation der Z7 und Z8 (der Salbe und des Aerosols) festgestellt.

Gleichzeitig verursachen D4, D8 und D23 Reizungen bereits bei den 2- bis 5mal niedrigeren Konzentrationen, was das Vorhandensein synergetischen Effekts beweist.

### Beispiel 4

Die Erforschung der spermiziden Wirkung erfolgte nach einem von der WHO empfohlenen Verfahren. Dafür wurden menschliche Spermatozoen mit verschiedenen Konzentrationen der AZ gemischt. Anschließend wurde die Beweglichkeit der Spermatozoen im Mikroskop geprüft. Es wurden auch andere Kennwerte (pH, Reduktion des Methylenblaus, Prüfung des Kontakts mit Zervikalschleim, Fruktosespiegel u. a. m.) erforscht. Es wurden insgesamt 17 Spermien von Männern im Alter von 18 bis 23 Jahren untersucht. Die Versuchsergebnisse haben gezeigt, dass die optimale Konzentration des angemeldeten Präparats, bei der die Beweglichkeit der Spermatozoen innerhalb von 20 s aufhört, 0,02 Gew.-% beträgt. Höhere Konzentrationen des Präparats verursachen nicht nur eine augenblickliche Unbeweglichkeit der Spermatozoen, sondern auch Spermatozoenlysis.

### Beispiel 5

Die vorbeugende Wirkung des angemeldeten Präparats bei Geschlechtskrankheiten wurde am Modell der experimentellen Syphilis bei Kaninchen erforscht. Die Versuche wurden an 56 Kaninchen der Chinchilla-Rasse durchgeführt. Die Kaninchen wurden mit einer frisch zubereiteten Aufschwemmung von blassen Spirochäten (Nichols-Stamm und ZKVI-8 [Zentrales Institut für Haut- und Geschlechtskrankheiten]) mittels eines epikutanten Verfahrens angesteckt. 30, 60, 120, 180 und 240 Minuten nach der Ansteckung wurde die Haut des betroffenen Bereichs mit Z6 oder D23 behandelt. Die Kaninchen aus der Vergleichsgruppe wurden mit destilliertem Wasser behandelt. Alle Tiere standen unter klinischer und serologischer Kontrolle im Laufe von 6 bis 12 Monaten. Für die Kontrolle wurden die Wassermann-Reaktion (RW), der Treponemen-Immobilisierungstest und die Immunofluoreszenz-Reaktion verwendet. Sie wurden 1,5 Monate nach der Ansteckung alle 2 - 3 Monate vorgenommen. Es wurden ebenfalls die Verfahren der Passivierung von Lymphknoten und der Reinfektion der Hodensackhaut mit der Suspension von Spirochäten aus dem gleichen Stamm benutzt. Die Versuchsergebnisse zeigten, dass die Bearbeitung von Versuchskaninchen mit Z6 sowie D23 innerhalb von 30 Minuten bis 2,5 Stunden ab dem Zeitpunkt der Ansteckung zum 100%igen Schutz der Tiere gegen Syphilis führt. Zugleich ergibt für D23 die Konzentration unter 0,01% kein 100%iges Ergebnis nach 30 Minuten ab dem Zeitpunkt der Ansteckung.

### Beispiel 6

Die Heilwirkung an Modellen von eitrigen Wunden und Peritonitis bei Versuchstieren (Ratten, Kaninchen, Meerschweinchen, Hunden, insgesamt über 100 Tiere) wurde gemäß allgemein anerkannter Verfahren erforscht. Dabei wurden bakteriologische, morphologische, histologische und immunologische Untersuchungsverfahren eingesetzt.

Die erhaltenen Angaben zeigten eine hohe therapeutische Aktivität der AZ. Die AZ wird als Wasserlösung, Salbe und Aerosol bei eitrigen Entzündungserkankungen und Peritonitis bei Versuchstieren angewendet. Die AZ erwies sich als ein viel wirksameres Präparat bei der Behandlung eitriger Wunden im Vergleich zu dem Prototyp und reinen Zutaten (Tab. 3).

**Tabelle 3**

| Wirkung der AZ auf die Heilung eitriger Wunden bei Versuchstieren (Ratten, Meerschweinchen, Kaninchen) im Vergleich zum Prototyp und reinen Zutaten | | |
|---|---|---|
| (*in Tagen*) | | |
| ***Tiergruppen*** | ***Anfang der Regeneration*** | ***Vollkommene Heilung*** |
| Kontrolle (Behandlung der Wunden mit destilliertem Wasser) | 7,2 ± 1,1 | 20,0 ± 3,5 |
| Behandlung mit D23 | 3,5 ± 0,7 | 12,7 ± 1,3 |
| Behandlung mit Z7 | 2,3 ± 0,3 | 9,4 ± 0,9 |
| Behandlung mit Z8 | 2,1 ± 0,3 | 9,2 ± 0,9 |
| Behandlung mit Z10 | 2,2 ± 0,3 | 9,3 ± 0,9 |
| 0,1 % Wasserlösung AO | 6,1 ± 1,1 | 18,0 ± 3,5 |
| 0,1 % Aerosol AA | 5,2 ± 1,3 | 17,0 ± 3,1 |

### Beispiel 7

Die Heilwirkung des angemeldeten Präparats an Verbrennungsmodellen bei Mäusen wurde untersucht. Die Kontakt-Hitzeverbrennung wurde auf der depilierten Dorsalfläche des Rückens der Mäuse mittels einer handelsüblichen Einrichtung bewirkt. Die Behandlung wurde 24 Stunden nach der Verbrennung begonnen. Dabei wurden eine Salbe und ein Aerosol (Z7 und Z8) benutzt. Zum Vergleich wurden auch D6 und D17 gemäß dem Prototyp angewendet. Die erfassten Ergebnisse sind in der Tabelle 4 angeführt.

Aus den Angaben der Tabelle 4 folgt, dass die zuverlässige Verminderung der Wundfläche bei Behandlungen mit Z7 und Z8 nach acht Tagen beginnt. Dies ist vier Tage früher als bei D6 und D17. Es kann behauptet werden, dass diese Reduzierung auf synergetischen Effekt zurückzuführen ist.

**Tabelle 4**

| Ergebnisse der Planimetrie von Verbrennungswunden, die mit Salbe und Aerosol behandelt wurden | | | | |
|---|---|---|---|---|
| ***Tage*** | ***Fläche der Verbrennungswunden, qcm*** | | | |
| | ***D6 und D17*** | | **Z7** | **Z8** |
| 2 | 0,72±0,05 | | 0,80 ± 0,06 | 0,99±0,08 |
| 4 | 0,77±0,66 | | 0,85 ± 0,07 | 0,81±0,09 |
| 6 | 0,80±0,05 | | 0,85±0,09 | 0,62±0,06 |
| 8 | 0,78±0,10 | | 0,83±0,10 | 0,76±0,07 |
| 12 | 0,81±0,05 | | 0,70±0,06 | 0,24±0,04 |
| 15 | 0,36±0,05 | | 0,18±0,03 | 0,14±0,03 |
| 18 | 0,14±0,04 | | 0,08±0,02 | 0,03±0,017 |
| 20 | 0,03±0,017 | | 0,012±0,002 | 0,01±0,001 |

### Beispiel 8

Die Wirkung bei eitrigen und entzündlichen Affektionen der Hornhaut wurde untersucht. Die Versuche wurden an 16 Kaninchen der Chinchilla-Rasse durchgeführt. Das Gewicht der Kaninchen betrug 2 - 2,5 kg. Die eitrigen und entzündlichen Beeinflussungen der Hornhaut wurden mittels der Verabreichung einer Suspension einer 24-stündigen Kultur des Staphylococcus-aureus-Stamms 209 P in die Hornhaut verursacht. Am 2. bis 4. Tag entstanden auf der Hornhaut von 13 Kaninchen Geschwüre. Die Geschwüre hatten eine Abmessung von 3 x 6 mm und wiesen auf ihrem Boden eine eitrige Absonderung auf. Es wurden gleichzeitig Entzündungsreaktionen beobachtet: Konjunktivitis, Blepharospasmus.u. a. m.

Die für den Versuch ausgewählten Kaninchen wurden in drei Gruppen eingeteilt. Die Tiere aus der ersten Gruppe wurden mit Z2 behandelt. Die Tiere aus der zweiten Gruppe wurden mit Augentropfen nach D7 und D15 (Augentropfen nach dem Prototyp, ATP) behandelt. In die betroffenen Augen der Tiere aus der dritten Gruppe, der Vergleichsgruppe, wurden Tropfen einer isotonischen Lösung von Natriumchlorid eingeträufelt. Die Präparate wurden täglich dreimal am Tag im Laufe des ganzen Versuchs eingeträufelt.

Alle Tiere wurden täglich beobachtet. Dabei wurde der Änderungsverlauf in der Hornhaut der Versuchs- und der Vergleichstiere nach dem früher beschriebenen Verfahren geprüft. Außerdem wurden Einimpfungen der Ausspülungen von der Hornhaut der Kaninchen in Fleischbrühe-Pepton-Nährböden angelegt, um das Vorhandensein des S.-aureus-Stamms 209 P festzustellen. Die Ergebnisse dieser Versuche sind in der Tabelle 5 angeführt.

**Tabelle 5**

| Ergebnisse der Behandlung von eitrigen und entzündlichen Affektionen der Hornhaut bei Kaninchen | | | | | | | | | | | | | | |
|---|---|---|---|---|---|---|---|---|---|---|---|---|---|---|
| *Merkmal der therapeutischen Wirkung* | *Tier gruppen-Nr.* | *Feststellungshäufigkeit von Merkmalen der therapeutischen Wirkung in der Hornhaut (in* %) *nach der Überwachungszeit (Tage):* ** | | | | | | | | | | | | |
| | | 2 | 3 | 4 | 5 | 6 | 7 | 8 | 10 | 11 | | 12 | 13 | 14 |
| Abwesenheit der eitrigen Absonderungen in den Augen | 1 | 50 (10) | 90 (10) | 100 | dito | dito | dito | dito | dito | Dito | | dito | dito | dito |
| | 2 | 12 (9) | 12 (9) | (10) | 88 (9) | 100 (9) | dito | dito | dito | dito | | dito | dito | dito |
| | 3 | 0 (6) | 0 (6) | 55 (9) 0 (6) | 0 (6) | 0 (6) | 16 (6) | 50 (6) | 66 (6) | 66 (6) | | 83 (6) | 83(6) | 100(6 |
| Abwesenheit von S. aureus in den eitrigen Absonderungen der Augen | 1 | 0 (10) | 9 (10) | 100 | dito | dito | dito | dito | dito | Dito | | dito | dito | dito |
| | 2 | (9) | 44 (9) | (10) | 88 (9) | 100 (9) | dito | dito | dito | dito | | dito | dito | dito |
| | 3 | 0 (6) | 0(6) | 66 (9) | 0 (6) | 0 (6) | 16 (6) | 50 (6) | 66(6) | 66 (6) | | 83 (6) | 83(6) | 100(6 |
| | | | | 0 (6) | | | | | | | | | | |
| Resorption des Eiterinfiltrats | 1 | 0 (10) | 40 (10) | 70(10) | 100 | dito | dito | dito | dito | Dito | | dito | dito | dito |
| | 2 | 0 (9) | 0 (9) | 11 (9) | (10) | 55 (9) | 66 (9) | 88 (9) | 100 (9) | dito | | dito | dito | dito |
| | 3 | 0 (6) | 0 (6) | 0 (6) | 44 (9) | 0(6) | 0(6) | 16 (6) | 16 (6) | 33(6) | | 33 (6) | 66 (6) | 66 (6) |
| | | | | | 0 (6) | | | | | | | | | |
| Epithelisierung | 1 | 20 (10) | 30 (10) | 30 (10) | 50 (10) | 80 (10) | 80 (10) | 8 (10) | 100 | Dito | | dito | dito | dito |
| | 2 | 0 (9) | 0 (9) | 0 (9) | 11 (9) | 11 (9) | 22 (9) | 22 (9) | (10 | 33 (9) | | 100 (9) | dito | dito |
| | 3 | 0 (6) | 0 (6) | 0 (6) | 0 (6) | 0 (6) | 0 (6) | 0 (6) | 33 (9) | 16 (6) | | (9) | 33 (6) | 50 (6) |
| | | | | | | | | | 0 (6) | | | 33 (6) | | |

| | | | | | | | | | | | | | | |
|---|---|---|---|---|---|---|---|---|---|---|---|---|---|---|
| Anmerkung: 1 Tiergruppe, die mit Z2 behandelt wurde; 2 Tiergruppe, die mit ATP behandelt wurde; 3 Vergleichs-Tiergruppe. (**) - In Klammern die Anzahl der untersuchten Augen. | | | | | | | | | | | | | | |

Die hier angeführten Angaben zeugen davon, dass die eitrigen Aussonderungen und die Nachweise von S. aureus am 4. Tag bei allen mit Z2 behandelten Tieren und am 6. Tag bei allen mit ATP behandelten Tieren aufgehört haben. Gleichzeitig wurden die eitrigen Aussonderungen bei allen nicht behandelten Tieren während der gesamten Versuchszeit beobachtet.

Die Resorption des Eiterinfiltrats begann und endete 3 Tage schneller bei allen Tieren, die mit Z2 behandelt wurden, am 3. Tag und am 5. Tag ab dem Anfang der Behandlung bei allen mit ATP behandelten Tieren. Bei allen Tieren, welche mit Z2 und ATP behandelt wurden, betrug die Zeitdauer vom Anfang bis zum Ende der Resorption des Eiterinfiltrats jeweils 4 Tage und 10 Tage ab Anfang der Behandlung. Die Epithelisierung der Hornhaut begann bei allen Tieren etwa am 10. Tag und etwa am 16. Tag ab dem Anfang der jeweiligen Behandlung mit Z2 und ATP.

Somit kann aus den oben angeführten Angaben geschlussfolgert werden, dass Z2 ein hocheffektives Mittel bei der Behandlung von eitrigen Entzündungen der Hornhaut darstellt. Dabei ist der Heileffekt der 22 höhe als der von ATP (Augentropfen nach dem Prototyp), die gewerblich hergestellt werden (Handelsname *"Okomistin").*

### Beispiel 9

Die entzündungshemmende Wirkung wurde untersucht. Dabei wurde die rationale Zusammensetzung der kombinierten Salbe zur Außenbehandlung der Allergodermatosen eingesetzt, die Z3 und 0,025% Betamethason enthält. Die gleiche Zusammensetzung mit dem Prototyp ergibt keine homogene Konsistenz und kann nicht als Arzneimittel angewendet werden.

Deshalb wurde die entzündungshemmende Wirkung am Modell des Aerosil-Ödems bei Ratten untersucht. Dabei wurde deren Wirkung mit der Wirkung der allgemein zu diesem Zweck angewandten Salbe "Lorinden S" und einer Vergleichsgruppe (nicht behandelt) vergleichen. Es wurde festgestellt, dass die Ausprägung des Ödems bei Ratten, die mit Z3 behandelt wurden, 2,67mal geringer als bei Vergleichstieren und 1,14mal geringer als beim Vergleichspräparat ist. Die Versuchsergebnisse sind in der Tabelle 6 dargestellt.

**Tabelle 6**

| Entzündungshemmende Wirkung der Salben | | | |
|---|---|---|---|
| Salbe | Anzahl der Ratten | Zuwachs der Rattenfüße, g | Inhibitorwirkung, % |
| Salbe auf der Basis des angemeldeten Präparats | 12 | 0,124±0,03 | 62,4 |
| Salbe "Lorinden S" | 12 | 0,141±0,01 | 57,0 |
| Vergleichsgruppe (nicht behandelt) | 12 | 0,331±0,02 | - |

Die in der Tabelle 6 angeführten Angaben zeugen davon, dass die Salbe Z3 eine besonders entzündungshemmende Wirkung aufweist. Sie reduziert das Aerosil-Ödem im Durchschnitt um 62,4 %, und ihre Effizienz übertrifft etwas die der vergleichbaren Salbe "Lorinden S".

### Beispiel 10

Untersuchungen der antiseptischen Eigenschaften der Suppositorienmasse.

Die antibakterielle Wirksamkeit von Z11 wurde im Vergleich mit der Wirksamkeit der Zutaten während der *"in vitro*"-Versuche mittels Diffusion in die festen Nährböden untersucht.
Während der Experimentalforschungen wurden als Test-Mikroorganismen folgende ausgewählt:
■ grampositive Kokken (Staphylococcus aureus ATCC 6538);
■ hefeartige Pilze (Candida albicans ATCC 885-653).

Um die antibakterielle Wirksamkeit zu untersuchen, wurde als Testkultur der Vergleichsstamm Staphylococcus aureus ATCC 6538 verwendet. Dieser Stamm ist vom ukrainischen D. K. Sabolotny-Institut für Mikrobiologie und Virusologie erhalten worden.
Für die Untersuchung der antifungalen Wirksamkeit wurde als Testkultur der ebenda erhaltene Vergleichsstamm Candida albicans ATCC 885-653 benutzt. Bevor die Versuche vorgenommen wurden, wurde jede Kultur auf Reinheit geprüft. Außerdem wurden je nach Kultur ihre Standard-Eigenschaften in Bezug auf morphologische und Kulturmerkmale geprüft.

Die antibakterielle Wirksamkeit wurde mittels Diffusion in den Agar folgendermaßen festgestellt: Die Nährböden wurden geschmolzen, bis zu einer Temperatur von 45°C gekühlt und mit den Aufschwemmungen der Testkulturen kontaminiert.
Die mikrobielle Beladung betrug ca. 1 x 10⁷ KBE/ml.
Je 20 ml des mit den Mikroben kontaminierten Mediums wurden in Petri-Schalen gegossen. Danach wurde das Medium erhärten gelassen. In der Schicht des Nähragars wurden Dellen mit einem Durchschnitt von 8 mm bereitgestellt.
Das zu untersuchende Präparat Z11 wurde bei einer Temperatur von 60°C geschmolzen und je 0,1 ml in die vorher vorbereiteten Dellen gegeben.

Zum Vergleich wurden Suppositorien mit 0,07% von D20 (D23) bzw. 0,07% AO bzw. 0,07% AA zubereitet.
Zum Vergleich wurden Wasserlösungen von Myramistin-Subs-tanz (D23) in den den zu prüfenden Präparaten entsprechenden Konzentrationen untersucht. Einimpfungen wurden angelegt und in den Thermostaten im Laufe von 24 Stunden bei einer Temperatur von 35°C inkubiert.
Nachdem die Inkubationszeit abgelaufen war, wurde der Durchschnitt der Stellen abgemessen, auf welchen die Mikroorganismen kein Wachstum zeigten (Hemmzonen). Die Dellen mit dem Präparat wurden ringsherum bis zu einer Genauigkeit von 0,1 mm bemessen. Jeder Versuch wurde dreimal vorgenommen.
Die Ergebnisse der antibakteriellen Wirksamkeit der zu untersuchenden Präparate wurden während des *"in vitro*"-Versuchs mittels Diffusionsverfahrens ermittelt und sind aus der Tabelle 7 ersichtlich.

**Tabelle 7**

| Bezeichnung des Präparates | Suppositorien | | Substanz (Myramistin) | |
|---|---|---|---|---|
| | Durchschnitt der Hemmzonen, mm | | Durchschnitt der Hemmzonen, mm | |
| | Staphylococcus aureus | Candida albicans | Staphylococcus aureus | Candida albicans |
| Myramistin, Wasserlösung 0,07% | - | - | 9,6 | 9,0 |
| Suppositorien D23 | 5,6 | 6,1 | | |
| AA | 4,3 | 5,2 | | |
| Z11 | 14,3 | 13,2 | | |
| AO | 1,2 | 2,1 | | |

Aus der Tabelle 7 ist der synergetische Effekt der angemeldeten Zusammensetzung ebenfalls ersichtlich.

## Patentansprüche

1. Arzneimittel, einschließlich Benzyldimethyl(3-[myristoylamino]-propyl)-ammoniumchlorid in Form von Monohydrat oder In nichthydratisierter Form,
**dadurch gekennzeichnet,**
**dass** es zusätzlich Dimethyl(3-[myristoylamino]-propyl)-aminoxid und/oder Dimethyl(3-[myristoylamino]-propyl)-amin im geeigneten pharmazeutischen Lösungsmittel enthält.

2. Arzneimittel nach Anspruch 1,
**dadurch gekennzeichnet,**
**dass** es zusätzlich ein Lokalanästhetikum aus folgender Hydrochlorid-Reihe enthält: Lidocain, Bupivacain, Pyromecain oder Trimecain in einer Menge von 0,1 - 5,0 Gew.-%.

3. Arzneimittel nach Anspruch 1,
**dadurch gekennzeichnet,**
**dass** es zusätzlich Natriumchlorid in einer Menge von 0,6 - 1 ,0 Gew.-% enthält.

4. Arzneimittel nach Anspruch 1,
**dadurch gekennzeichnet,**
**dass** es zusätzlich ein Antiödemmittel aus der Reihe Xylometazolin, Oxymetazolin, Naphazolin, Phenylephrin, Phenylpropanolamin oder Pseudoephedrin in einer Menge von 0,01 - 2,0 Gew.-% enthält.

5. Arzneimittel nach Anspruch 1,
**dadurch gekennzeichnet,**
**dass** es zusätzlich Kortikosteroid aus der Reihe Triamcinolon, Betamethason oder Fluocinolone acetonid, Hydrocortison, Halometason oder Dexamethason in einer Menge von 0,1 - 3,0 Gew.-% enthält.

6. Arzneimittel nach den Anspruch 1,
**dadurch gekennzeichnet,**
**dass** es zusätzlich Antiseptika und/oder virentötende Präparate aus der Reihe Metronidazol, Clotrimazol, Ketooonazole, Aciclovir oder Rimantadin in einer Menge von 0,01 - 5,0 Gew.-% enthält.

7. Arzneimittel nach den Ansprüchen 1 bis 6.
**dadurch gekennzeichnet,**
**dass** es als pharmazeutisches Lösungsmittel Alkohol und/oder Wasser enthält.

8. Arzneimittel nach den Ansprüchen 1 bis 6,
**dadurch gekennzeichnet,**
**dass** es als pharmazeutisches Lösungsmittel einen beliebigen flüssigen, gelartigen Salben-, Wasch- oder Festgrundstoff pflanzlicher, tierischer oder synthetischer Herkunft enthält.

9. Arzneimittel nach einem der Ansprüche 1 bis 8,
zur Verwendung bei der Behandlung und Vorbeugung von eitrigen Entzündungskrankheiten mit verschiedener Ätiologie und Lokalisierung.

## Claims

1. A pharmaceutical product, including benzyldimethyl(3-[myristoylamino]propyl)ammonium chloride in the form of monohydrate or in nonhydrogenated form,
**characterized in that**
it additionally contains dimethyl(3-[myristoylamino]propyl)amine oxide and/or dimethyl(3-[myristoylamino]propyl)amine in an appropriate pharmaceutical solvent.

2. The pharmaceutical product of claim 1,
**characterized in that**
it additionally contains a local anesthetic selected from the following hydrochloride series: lidocaine, bupivacaine, pyromecaine or trimecaine, in an amount of from 0.1 to 5.0 percent by weight.

3. The pharmaceutical product of claim 1,
**characterized in that**
it additionally contains sodium chloride in an amount of from 0.6 to 1.0 percent by weight.

4. The pharmaceutical product of claim 1,
**characterized in that**
it additionally contains an antiedemic agent selected from the series consisting of xylometazoline, oxymetazoline, naphazoline, phenylephrine, phenylpropanolamine or pseudoephedrine, in an amount of from 0.01 to 2.0 percent by weight.

5. The pharmaceutical product of claim 1,
**characterized in that**
it additionally contains corticosteroid selected from the series consisting of triamcinolone, betamethasone or fluocinolone acetonide, hydrocortisone, halomethasone or dexamethasone, in an amount of from 0.1 to 3.0 percent by weight.

6. The pharmaceutical product of claim 1,
**characterized in that**
it additionally contains antiseptics and/or virucidal preparations selected from the series consisting of metronidazole, clotrimazole, ketoconazole, acyclovir or rimantidine, in an amount of from 0.01 to 5.0 percent by weight.

7. The pharmaceutical product of one of claims 1-6,
**characterized in that**
it contains alcohol and/or water as the pharmaceutical solvent.

8. The pharmaceutical product of one of claims 1-6,
**characterized in that**
it contains an arbitrary liquid, gel-like ointment base, detergent base or solid base of vegetable, animal or synthetic origin as the pharmaceutical solvent.

9. The pharmaceutical product of one of claims 1-8,
for use in the treatment and prevention of purulent inflammatory diseases of varying etiology and localization.

## Revendications

1. Médicament, comprenant du chlorure de benzyldiméthyl(3-[myristoylamino]-propyl)-ammonium sous forme de monohydrate ou sous forme non hydratée,
**caractérisé en ce**
**qu'**il contient en outre du diméthyl(3-[myristoylamino]-propyl)-amine-oxyde et/ou de la diméthyl(3-[myristoylamino]-propyl)-amine dans un solvant pharmaceutique convenable.

2. Médicament selon la revendication 1,
**caractérisé en ce**
**qu'**il contient en outre un anesthésique local choisi dans la série suivante de chlorhydrates : lidocaïne, bupivacaïne, pyromécaïne ou trimécaïne en une quantité de 0,1 - 5,0 % en poids.

3. Médicament selon la revendication 1,
**caractérisé en ce**
**qu'**il contient en outre du chlorure de sodium en une quantité de 0,6 - 1,0 % en poids.

4. Médicament selon la revendication 1,
**caractérisé en ce**
**qu'**il contient en outre un anti-oedémateux choisi dans la série constituée par la xylométazoline, l'oxymétazoline, la naphazoline, la phényléphrine, la phénylpropanolamine ou la pseudo-éphédrine, en une quantité de 0,01 -2,0 % en poids.

5. Médicament selon la revendication 1,
**caractérisé en ce**
**qu'**il contient en outre un corticostéroïde choisi dans la série constituée par la triamcinolone, la bétaméthasone ou la fluocinolone acétonide, l'hydrocortisone, l'halométasone ou la dexaméthasone, en une quantité de 0,1 - 3,0 % en poids.

6. Médicament selon la revendication 1,
**caractérisé en ce**
**qu'**il contient en outre des antiseptiques et/ou des préparations virucides choisis dans la série constituée par le métronidazole, le clotrimazole, le kétoconazole, l'aciclovir ou la rimantadine, en une quantité de 0,01 - 5,0 % en poids.

7. Médicament selon les revendications 1 à 6,
**caractérisé en ce**
**qu'**il contient comme solvant pharmaceutique de l'alcool et/ou de l'eau.

8. Médicament selon les revendications 1 à 6,
**caractérisé en ce**
**qu'**il contient comme solvant pharmaceutique une base de pommade liquide, gélatineuse, lavante ou solide quelconque d'origine végétale, animale ou synthétique.

9. Médicament selon l'une quelconque des revendications 1 à 8,
pour utilisation dans le traitement et la prévention de maladies inflammatoires purulentes à localisation et étiologie variées.
